# EUROPEAN PATENT SPECIFICATION

(11) **EP 3 730 062 B1**
(45) Date of publication and mention of the grant of the patent: **12.03.2025**
(21) Application number: 18892834.5
(22) Date of filing: 09.11.2018
(51) Int. Cl.: A61B 90/00, A61B 34/37

(54) **SURGICAL ROBOT SYSTEM AND SURGICAL INSTRUMENT THEREOF**
CHIRURGISCHES ROBOTERSYSTEM UND CHIRURGISCHES INSTRUMENT DAFÜR
SYSTÈME DE ROBOT CHIRURGICAL ET INSTRUMENT CHIRURGICAL ASSOCIÉ

(30) Priority: 21.12.2017 CN 201711394632
(43) Date of publication of application: 28.10.2020
(73) Proprietor: Shanghai Microport Medbot (Group) Co., Ltd., Shanghai 201203 (CN)
(72) Inventor: LI, Tao, Shanghai 201203 (CN); ZHU, Xiang, Shanghai 201203 (CN); HE, Chao, Shanghai 201203 (CN)
(74) Representative: Patentship Patentanwaltsgesellschaft mbH
(86) International application number: PCT/CN2018/114902
(87) International publication number: WO 2019/120005

(56) References cited:
- EP-A2- 0 177 919
- EP-B1- 0 177 919
- CN-A- 101 325 920
- CN-A- 105 796 043
- CN-A- 108 042 162
- CN-U- 206 151 580
- US-A- 5 391 144
- US-A1- 2007 151 390
- US-A1- 2010 313 679
- US-A1- 2012 265 102
- US-A1- 2013 197 538

## Description

### TECHNICAL FIELD

The present application relates to the technical field of medical device and, more specifically, to a surgical robot system and a surgical instrument thereof.

### BACKGROUND

Minimal invasive surgery is accepted by more and more patients because of its advantages of small wounds and rapid recovery, and more and more surgical modes are applied in minimal invasive surgeries gradually progressed from traditional open surgery. At the same time, the continuous advancement of surgical methods has also greatly promoted the development and progress of corresponding surgical devices. Early handheld surgical devices have been continuously replaced by mechanized and intelligent surgical devices. As the most advanced surgical device in the contemporary era, surgical robot systems are constantly impacting people's medical ideas, and various minimal invasive surgical robot systems with different functions are constantly emerging.

In minimal invasive surgery, laparoscopic surgery has been basically popular because of its early realization. Among existing laparoscopic surgical robot systems, the DaVinci system has been recognized as one of the most outstanding ones in the world, and the use of the DaVinci system almost covers Europe and America, which reflects that it has absolute advantages. The basic control concept of the DaVinci surgical robot system is the control mode of the master-slave remote control operation. The doctor controls the surgical instruments of the bedside robot through the master manipulator of the master console. In general, the bedside robot has multiple robotic arms capable of holding surgical instruments and an endoscope. However, the surgical instruments of the surgical robot system are different from traditional surgical instruments, and they have features of more automatic and intelligent.

The surgical instruments of the surgical robot system generally contain four degrees of freedom: rotation, swing, pitching, and opening/closing, so that they can best simulate the acting of human hands. When the doctor operates the robot, the operations of the surgical instrument like operations performed by the doctor's own hands. Moreover, surgical instruments are more flexible than human hands and can perform operations that human hands cannot do. However, surgical instruments in such systems as the inventor knows, are still suffering from a number of deficiencies, the major ones of which are as follows:
(1) Some existing surgical robots are absent of force feedback mechanisms, leaving surgical instruments therein unable to feed back their operating environment and status to the doctor during an operation. Consequently, the doctor cannot perceive any interference to the surgical instruments from outside the field of vision, or any of the surgical instruments touch with a certain body tissue, but the doctor is not aware of that, which may cause an undesired operation. This will significantly affect the doctor's feeling during the manipulation and the outcome of the operation or even lead to failure of the operation.
(2) Although some surgical robots include a force feedback mechanism, the structure of the force feedback mechanism is complicated. For example, the surgical robot with force feedback mechanism disclosed in Chinese patent publication CN101340850A is provided with a plurality of strain gauges arranged outside the distal end of the shaft connected to the end effector, for detecting the strain parallel to the longitudinal axis of the shaft, and then corresponding forces can be calculated based on the strain. The above surgical robot employs an indirect force feedback structure to realize the force feedback mechanism of the surgical instrument. The strain gauges themselves are not directly connected or in contact with the end effector. A more complicated algorithm is needed to convert the strain measured by the strain gauge into forces applied to the surgical instruments, and the measurement accuracy of the indirect force feedback mechanism is relatively low, which does not meet the design purpose of surgical instruments for surgical robots.
US 2010/313679 A1 discloses a modular force sensor which comprises a tube portion including a plurality of strain gauges, a proximal tube portion for operably coupling to a shaft of a surgical instrument and a distal tube portion for proximally coupling to a wrist joint coupled to an end portion. EP 0 177 919 A2 discloses a robot having a force sensor attached between a wrist portion and a hand portion. US 2013/197538 A1 discloses a robotic hand controller for enabling a user to perform an activity.

### SUMMARY OF THE INVENTION

The invention is set out in the appended set of claims.

It is an objective of some embodiments of the present application to overcome at least one of the problems of conventional mechanisms for measuring a contact force acting on a terminal of a surgical instrument, such as structural complexity, computational complexity and low accuracy, by providing a surgical robot system and a surgical instrument thereof.

According to an aspect of the present application, there is provided a surgical instrument, comprising a mechanical structural unit and a pressure sensor unit, wherein,
the mechanical structural unit comprises an instrument shaft and an end effector; the instrument shaft comprises a body and a connecting portion extending from a distal end of the body; the connecting portion comprises a first connector and a second connector that are radially distributed; the first connector is fixedly connected to the body of the instrument shaft, and the second connector is fixedly connected to the end effector;
the pressure sensor unit comprises a sensitive element disposed between the first connector and the second connector, and the sensitive element is configured to sense a force exerted by the connecting portion on the sensitive element to determine a Cartesian force received by the end effector.

Optionally, the first connector is a hollow support shaft, the second connector is a hollow base, the base is formed from an axial extension of a proximal end of the end effector, and the base is configured to sleeve with the support shaft.

Optionally, the first connector is an outer layer structure, the second connector is an inner layer structure, the outer layer structure is radially connected with the inner layer structure to form a groove in which the sensitive element is arranged.

Optionally, the groove has a U-shaped axial section.

Optionally, a size of an outer surface of the first connector is smaller than a size of an inner surface of the second connector; and
the size of the inner surface of the second connector is smaller than a sum of the size of the outer surface of the first connector and a radial dimension of the sensitive element.

Optionally, a size of an inner surface of the second connector is greater than a sum of a size of an outer surface of the first connector and a radial dimension of the sensitive element, and a filler is arranged between the first connector and the second connector to increase an elasticity between the first connector and the second connector.

Optionally, a size of an inner surface of the first connector is larger than a size of an outer surface of the second connector, and
the size of the inner surface of the first connector is smaller than a sum of the size of the outer surface of the second connector and a radial dimension of the sensitive element.

Optionally, a size of an inner surface of the first connector is larger than a sum of a size of an outer surface of the second connector and a radial dimension of the sensitive element, and a filler is arranged between the first connector and the second connector to increase an elasticity between the first connector and the second connector.

Optionally, the filler is made from rubber or silicone.

Optionally, the pressure sensor unit comprises one sensitive element, or comprises a plurality of sensitive elements, and the plurality of sensitive elements are distributed along a circumferential direction and/or an axial direction of the surgical instrument.

Optionally, the plurality of sensitive elements are distributed in a plurality of rows in the axial direction of the surgical instrument, and the sensitive elements in each of the rows are evenly distributed in the circumferential direction of the surgical instrument.

Optionally, each of the rows of sensitive elements is arranged staggeredly with an adjacent one of the rows of sensitive elements.

According to another aspect of the present application, there is provided a surgical robot system, comprising a slave device comprising:
a robotic arm; and
a surgical instrument;
wherein the robotic arm has a terminal detachably connected to the surgical instrument, and is configured to drive the surgical instrument to pivot about a remote center of motion.

Optionally, the surgical robot system further comprises a master device and a control unit, the master device comprising a force indicator;
wherein the control unit is communicatively connected to the master device and to the slave device, and the control unit is configured to obtain information about a Cartesian force received by the end effector from the sensitive element of the surgical instrument and transmits the information to the force indicator.

Optionally, the force indicator is a master manipulator provided with a motor, and the control unit is configured to issue a torque command to the motor of the master manipulator to enable an operator to feel a force acting on the terminal of the surgical instrument.

Optionally, the master manipulator further comprises a vibrating motor; when the force acting on the terminal of the surgical instrument exceeds a preset threshold, the control unit issues a vibration command to the vibrating motor of the master manipulator, notifying the operator about the excessive force acting on the terminal of the surgical instrument.

The surgical instrument according to the present application has a first connector and a second connector that are radially distributed, and a sensitive element of a pressure sensor unit is provided between the first connector and the second connector, and the sensitive element is configured to sense the force applied by the second connector onto the first connector, and according to the force information, the Cartesian force acting on the end effector of the surgical instrument can be determined.

In some embodiments, the pressure sensor unit is a strain pressure sensor, a piezoresistive pressure sensor, or a piezoelectrical pressure sensor. The sensitive element is disposed between the first connector and the second connector to receive the force at the connecting area. When the end effector of the surgical instrument is subjected to an external force (i.e., a Cartesian force), the force exerted by the second connector on the sensitive element and on the first connector deforms the sensitive element and generates deformation information. Further, through determining the pressure between the first connector and the second connector based on the deformation information, the Cartesian force acting on the end effector of the surgical instrument can be accurately and uniquely measured.

In particular, the distal end of the instrument shaft of the surgical instrument extends axially to form a double-layer and hollow support shaft. Preferably, the support shaft has a groove with a U-shaped axial cross-section. Due to the U-shaped thin wall of the support shaft, the thin-walled feature can further improve the accuracy in determining the force acting on the end effector of the surgical instrument.

Compared with the conventional solutions using a motor output to calculate the force acting on the end effector of the surgical instrument, the surgical instrument of the present application has advantages of both a simpler force transmission path and higher force measurement accuracy. Moreover, the force acting on the terminal of the surgical instrument can be determined in an easier manner without requiring additional components, providing for lower structural complexity of the surgical instrument and facilitating its assembly. Further, since minor changes are required in the surgical instrument, various existing surgical instruments after being modified with minor changes also can be suitably used in the surgical robot system proposed by the present application.

### BRIEF DESCRIPTION OF THE DRAWINGS

Fig. 1 is a structural schematic of a surgical robot system according to an embodiment of the application.
Fig. 2a is a structural schematic of a surgical instrument according to an embodiment of the application.
Fig. 2b is a partial enlarged view of the surgical instrument shown in Fig. 2a.
Fig. 3a schematically shows a structure of a terminal of a surgical instrument with a deformable portion not integratedly formed according to an embodiment of the application.
Fig. 3b schematically shows a structure of a terminal of a surgical instrument with a deformable portion not integratedly formed according to another embodiment of the application.
Fig. 4a schematically shows a structure of a terminal of a surgical instrument with a deformable portion integratedly formed according to a further embodiment of the application.
Fig. 4b is a cross-sectional view of the terminal of the surgical instrument shown in Fig. 4a.
Fig. 4c is a partially enlarged view of a terminal of the surgical instrument shown in Fig. 4b.

List of reference signs:
1 denotes a surgical cart; 2 denotes a robotic arm; 3 denotes a surgical instrument; 301 denotes a power module; 302 denotes a mounting base; 303 denotes an instrument shaft; 304 denotes a force transmission mechanism; 305 denotes an end effector; 4 denotes an endoscope; 5 denotes a imaging system; 6 denotes a master manipulator; 7 denotes an armrest; 8 denotes a console base;
9 denotes a sensing device; 10 denotes a control unit; 310, 313 denotes a terminal base; 312, 315, 321 denotes a sensitive element; 311, 314 denotes a support shaft; 320 denotes a connecting portion; 323 denotes an outer layer structure; 324 denotes an inner layer structure; 322 denotes a groove.

### DETAILED DESCRIPTION

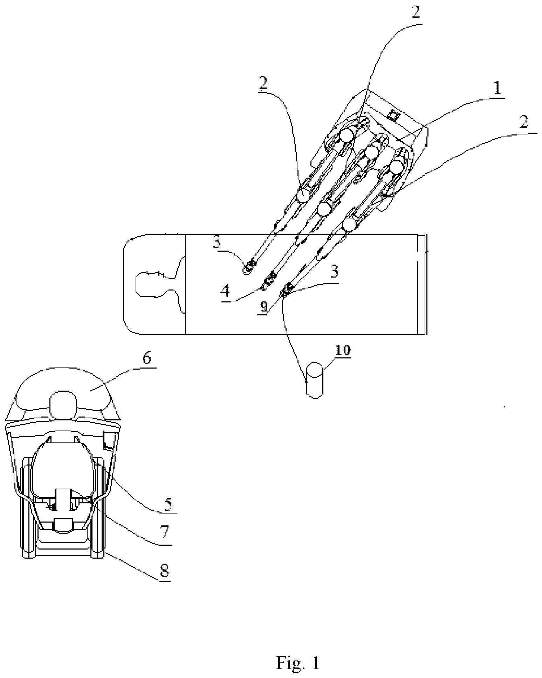

The above and other objectives, features and advantages of the present application will become more apparent from the following detailed description of the proposed surgical robot system and surgical instrument thereof, which is to be read in connection with Figs. 1 to 4. Note that the figures are much simplified and may not be drawn to scale, and the only purpose of them is to facilitate easy and clear explanation of the disclosed embodiments. As used herein, a "trailing end", "terminal" or "distal end" refers to an end farther way from an operator and closer to a patient, while a "leading end" or "proximal" refers to an end closer to the operator and farther way from the patient. As used in present disclosure, the meaning of "a" "an" and "the" include singular and plural references, unless the context clearly dictates otherwise.

The surgical robot systems according to these embodiments described below are able to measure a radial force and/or an axis force acting on the terminal of the surgical instrument.

Fig. 1 is a structural schematic of a surgical robot system. The surgical robot system includes a slave device including a surgical cart 1, robotic arms 2, a surgical instrument 3, an endoscope 4. As a base of the whole slave device, the surgical cart 1 supports all the other components of the slave device described above. Meanwhile, the surgical cart 1 is moveable on the ground to allow the slave device to approach or leave a patient.

The robotic arm 2 with multiple degrees of freedom is mounted on the surgical cart 1 and configured to drive the surgical instrument 3 to pivot about a remote center of motion. When the surgical cart 1 moves to the vicinity of the patient, the robotic arm 2 may be adjusted so that the surgical instrument 3 arrives at a predetermined target surgical site. In other words, the remote center of motion is located around the surgical site by adjusting both the surgical cart 1 and the robotic arm 2. The surgical instrument 3 is detachably mounted at a terminal of the robotic arm 2 through a fixed connection or a movable connection. As an output of the slave device, the surgical instrument 3 will eventually enter into the patient's body at the surgical site so as to treat a target lesion.

The endoscope 4 is mounted at a terminal of one of the robotic arms 2 different from that on which the surgical instrument 3 is coupled and is configured to collect image information about the surgical environment. The image information may include, but is not limited to, the information about tissue around the lesion and that about a posture and position of the surgical instrument 3. When mounted on the robotic arm 2, the endoscope 4 may be communicatively connected to the master device as detailed below so as to enable real-time display of the information about the surgical environment collected thereby. The endoscope 4 may be three-dimensional or not, which is not limited by the present application.

With continued reference to FIG. 1, the surgical robot system further includes a master device which includes an imaging system 5, a master manipulator 6, an armrest 7 and a console base 8. During a surgical operation, with the information from the endoscope 4 being displayed by the imaging system 5, a doctor can observe motion of the surgical instrument 3 through the imaging system 5, and accordingly control the subsequent movement of the surgical instrument 3 by manipulating the master manipulator 6. The doctor may sit at a surgical console and, with the aid of the imaging system 5, observe the position and motion of the terminal of the surgical instrument in vivo. Based on the observations, the doctor can control a multi-dimensional movement (such as rotation, swing, pitching, and opening/closing) of the terminal by manipulating the master manipulator 6, thus allowing for a minimally invasive operation. The armrest 7 can support the doctor's arm so that the doctor can maintain a higher comfort when the surgical operation lasts for a long time. In addition, the armrest 7 can be raised and lowered to meet various needs of different doctors. The console base 8 serving as the basic structure of the master device can move freely on the ground, and it supports all the other structures of the master device described above.

The specific surgical operation of the surgical robot system is described as follows.

Firstly, the doctor control the surgical cart 1 and the console base 8 to push the slave device to the vicinity of the operating table where the patient lies, so that the slave device is in a better operation position, and to push the master device to a relatively better manipulation position, which is convenient for doctors to manipulate.

Then, through adjusting the mechanical arm 2, the surgical instrument 3 and the endoscope 4 are driven to the vicinity of the surgical incision.

After that, the surgical instrument 3 and the endoscope 4 are inserted into the patient's body through the incision on the patient;

Finally, the doctor observes the position and movement state of the end effector of the surgical instrument 3 in the patient's body through the stereoscopic imaging system 5, and adjusts the position and movement state of the end effector through the master manipulator 6, and then performs a minimal invasive surgery.

Apparently, the control made from the master manipulator 6 to the surgical instrument 3 is basic to the master/slave control in the surgical robot system. In order to better simulate the actual circumstances of a surgical operation, i.e., simulate the force acting on the surgical instrument 3 during the operation, it is desired that the surgical instrument 3 is able to feed back any force acting on it to the master manipulator 6, i.e., providing the surgical instrument 3 with force feedback capabilities, so that the doctor can adaptively adjust surgical operation. Therefore, the present application provides a surgical instrument equipped with a sensing device and a corresponding surgical robot system.

Specifically, the surgical instrument 3 further includes a sensing device 9 for sensing the force acting on the terminal of the surgical instrument 3. The surgical robot system further includes a control unit 10 for receiving and transmitting the information about the force acting on the surgical instrument 3 acquired by the sensing device 9. The control unit 10 communicatively connected to both the master and slave devices, for example, by wired or wireless connections. The control unit 10 is responsible for, based on a control strategy, processing data from the sensing device 9 and calculating various data required in the controlling. The control unit 10 is configured to transmit information about the force acting on the terminal of the surgical instrument 3 to a force indicator of the master device, so that the force acting on the terminal of the surgical instrument 3 can be perceived by the doctor.

The force indicator may be the imaging system 5, which can display the magnitude and direction of the force acting on the terminal of the surgical instrument 3. Alternatively, the force indicator may be the master manipulator 6 equipped with a motor. While the doctor manipulates the system, the control unit 10 may control the motor of the master manipulator 6 based on the information about the force acting on the terminal of the surgical instrument 3 and may exert a force acting onto the doctor. Apparently, the control made from the master manipulator 6 to the surgical instrument 3 is basic to the master/slave control in the surgical robot system. In order to better simulate the actual circumstances of a surgical operation, i.e., simulate the force acting on the surgical instrument 3 during the operation, it is desired that the surgical instrument 3 is able to feed back any force acting on it to the master manipulator 6, and provided with force feedback capabilities. After a force acting on the terminal of the surgical instrument 3 is determined based on the force data sensed by the sensing device 9, the control unit 10 can issue a torque command to the motor of the master manipulator 6, to enable the operator to perceive the force acting on the terminal of the surgical instrument 3. More preferably, the master manipulator 6 may be provided with a vibrating motor. In this case, when the force acting on the terminal of the surgical instrument 3, which is determined from the force data sensed by the sensing device 9, exceeds a preset threshold, the control unit 10 can issue a vibration command to the vibrating motor of the master manipulator 6, notifying the operator about the excessive force acting on the terminal of the surgical instrument 3.

In the present application, the surgical instrument includes a mechanical structural unit and a pressure sensor unit. The mechanical structural unit includes an instrument shaft and an end effector. The instrument shaft includes a body and a connecting portion extending from a distal end of the body. The instrument shaft is connected to the end effector through the connection portion, and the pressure sensor unit serves as the sensing device 9 of the present application, and the sensitive element thereof senses the force thereon applied by the connection portion (that is, the Cartesian force received by the end effector). Next, the implementation of the surgical instrument will be described in further detail.

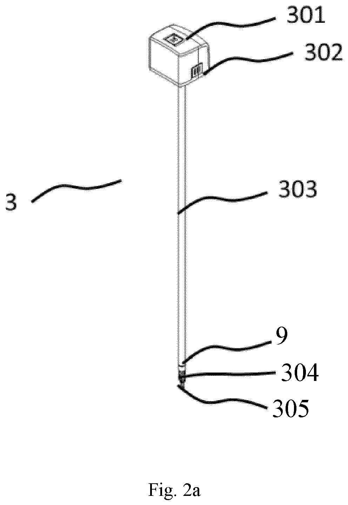

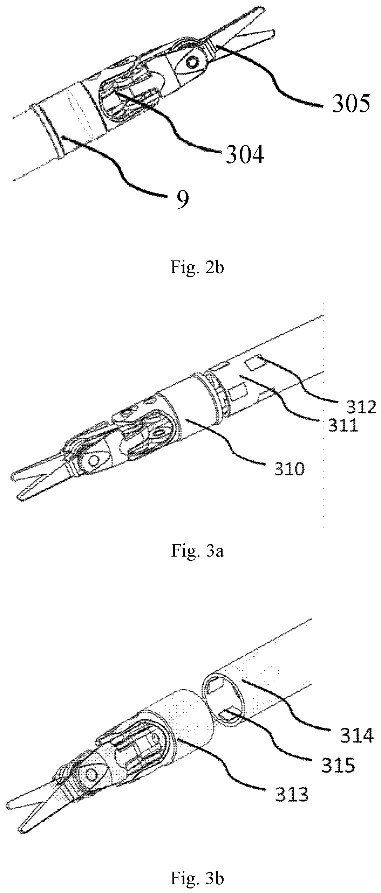

First, referring to Figs. 2a and 2b, Fig. 2a schematically shows a mechanical structural unit of a surgical instrument, and FIG. 2b shows a partially enlarged view of the mechanical structural unit of the surgical instrument shown in FIG. 2a. As shown, the mechanical structural unit of the surgical instrument 3 includes a power module 301, a mounting base 302, an instrument shaft 303, a force transmission mechanism 304 and an end effector 305.

The power module 301 is disposed at a proximal end of the instrument shaft 303, while the end effector 305 is disposed at a distal end of the instrument shaft 303. The power module 301 is configured to provide a driving force, which is transferred by the force transmission mechanism to the end effector 305, thus enabling the end effector 305 to perform a multi-dimensional rotational motion and/or opening/closing action, etc.

The power module 301 is detachably connected to an external motor, and configured to receive force from the external motor. Particularly, the power module 301 is connected to the motor through a reducer. The force output by the motor is increased by the reducer and then transmitted to end effector 305 through the power module 301 and the force transmission mechanism 304. For example, the force transmission mechanism 304 is a wire transmission, which includes a steel wire and a guide wheel. The steel wire is used to transmit force, and the guide wheel is used to adjust the extension direction of the steel wire. Specifically, the force transmission mechanism 304 goes through the instrument shaft 303 and is connected to the power module 301 and the end effector 305. The end effector 305 is configured to perform specific operations, such as scissoring, knotting and grabbing, to the lesion site in the patient's body. The present application is not limited to any particular type of the end effector 305 as it can be scissors, pliers, a probe or the like.

The mounting base 302 is detachably connected to the terminal of the robotic arm 2. Preferably, the power module 301 is accommodated in the mounting base 302. The proximal end of the instrument shaft 303 is connected to the mounting base 302, and the distal end is connected to the end effector 305. The instrument shaft 303 has a sufficient length so that the end effector 305 can treat a patient's site during the operation.

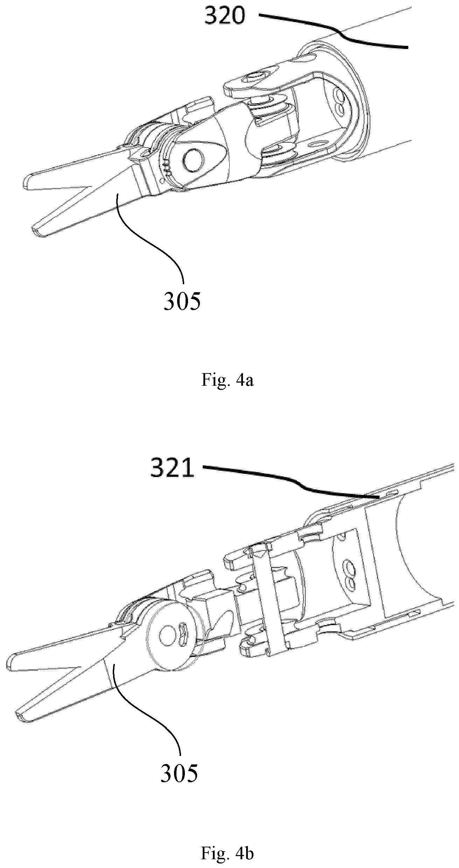

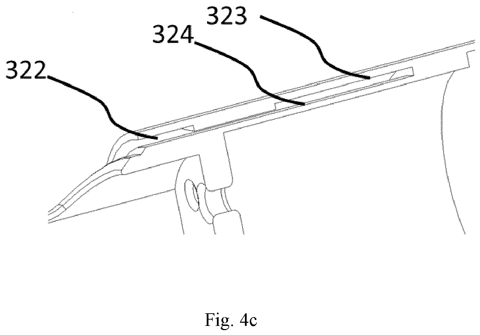

The instrument shaft 303 includes a body and a connecting portion 320 extending from the distal end of the body (see Fig. 4a). The connecting portion 320 includes a first connecting member and a second connecting member that are radially distributed, the first connecting member is fixedly connected to the body of the instrument shaft, and the second connecting member is fixedly connected to the end effector. Furthermore, the sensitive element of the pressure sensor unit is disposed between the first connection member and the second connection member, that is, located on the connection portion 320 of the instrument shaft 303, for sensing the force exerted thereon by the connection portion 320, thereby determining the force acting on the connection part 320 and even the overall stress of the end effector 305 (that is, the reactive force from the human tissue to the end effector 305). The connecting portion 320 can be formed separately from the body of the instrument shaft 303, that is, processed separately and then assembled together to become a whole. In addition, the sensitive element may be selected from strain gauges such as piezoelectric strain gauges, piezoresistive strain gauges, strain gauges, etc., to sense the force acting on the connecting portion 320. In some embodiments, the pressure sensor unit includes a circuit containing a sensitive element. When the sensitive element deforms due to force and the resistance changes, the current or voltage of the circuit also changes. Based on relationship between the calibrated current/voltage and pressure, the pressure sensor unit senses the pressure acting on the sensitive element.

For example, in the embodiment shown in FIG. 3a, the first connector and the second connector are designed as two separate members. Specifically, the second connector is a terminal base 310 connected to the proximal end of the end effector 305, and the terminal base 310 has a hollow structure to facilitate the passage of the aforementioned force transmission mechanism 304. The first connector is a support shaft 311 fixedly connected to the body of the instrument shaft 303. Similarly, the support shaft 311 also has a hollow structure to facilitate the passage of the force transmission mechanism 304. The diameter and material of the support shaft 311 may be the same as other parts of the instrument shaft 303, or may be different. Moreover, one or more sensitive elements 312 are attached to the outer surface (preferably an outer circular surface) of the support shaft 311. Preferably, a plurality of the sensitive elements 312 are evenly distributed along the circumferential direction of the support shaft 311. More preferably, the support shaft 311 is provided with a plurality of rows of sensitive elements 312 distributed uniformly in the axial direction, and each row of sensitive elements 312 and the adjacent row of sensitive elements 312 are staggered. In addition, the inner diameter of the terminal base 310 is larger than the outer diameter of the support shaft 311, and slightly smaller than the sum of the outer diameter of the support shaft 311 and the radial dimension of the sensitive element 312 to obtain an interference fit, so that the support shaft 311 can be inserted into the terminal base 310 and fixed with it. Here, the radial dimension of the sensitive element 312 is the thickness of the sensitive element 312 along the cross-sectional direction of the terminal base 310 or the support shaft 311. Before the use of the sensitive element 312, the measurement error of sensitive components due to interference fit can be removed through adjustment to the measurement reference. In other embodiments, the inner diameter of the terminal base 310 is slightly larger than the sum of the outer diameter of the support shaft 311 and the radial dimension of the sensitive element 312, that is, a clearance fit is formed between the two. If the base terminal 310 in clearance fit with the support shaft 311, a filler, such as an elastic rubber, silicone, is preferably provided in the clearance between the terminal base 310 and the support shaft 311, for the better elasticity of the connecting portion 320. Further, the base terminal 310 and the support shaft 311 are preferably coaxially arranged and in a clearance fit manner.

In the connection portion 320 formed by the terminal base 310 and the support shaft 311 being sleeved therein, the support shaft 311 is fixedly connected to the body of the instrument shaft 303, the terminal base 310 is fixedly connected to the end effector 305, and the sensitive element 312 is disposed between the support shaft 311 and the terminal base 310. When the end effector 305 of the surgical instrument 3 is subjected to Cartesian force and transmits the Cartesian force to the terminal base 310, the terminal base 310 exerts the Cartesian force on the sensitive element 312. This force is immediately sensed by the sensitive element 312. Therefore, through measuring the force acting on the terminal base 310 and the support shaft 311, the Cartesian force received by the surgical instrument terminal 305 can be accurately and uniquely measured, and moreover, measurement errors caused by changes in the structure of the surgical instrument terminal can be avoided.

Specifically, during an actual surgery, when the end effector 305 of the surgical instrument 3 is subjected to Cartesian force exerted by human tissue, the end effector 305 transmits the Cartesian force to the terminal base 310, and the terminal base 310 then exerts the Cartesian force onto the sensitive element 312 which is attached to the outer surface of the support shaft 311. Therefore, based on the force data obtained from sensing of the sensitive element 312, the Cartesian force received by the end effector 305 of the surgical instrument 3 can be perceived.

However, the sensitive element 312 is not limited to being attached to the outer surface of the support shaft 311, but can also be attached to the inner surface (preferably an inner circular surface) of the terminal base 310. In this case, the inner diameter (i.e., the inner diameter of the terminal base 310 minus the radial dimension of the sensitive element 312) of the terminal base 310 to which the sensitive element 312 has been attached may be slightly smaller than the outer diameter of the support shaft 311 to obtain an interference fit.

Different from the foregoing embodiment, the first connector of the connection portion 320 is sleeved over the second connector, see Fig. 3b for details. In the embodiment shown in FIG. 3b, the second connector is a terminal base 313 connected to the proximal end of the end effector 305, and the terminal base 313 has a hollow structure to facilitate the passage of the force transmission mechanism 304. The first connector is a support shaft 314 fixedly connected to the body of the instrument shaft 303. Similarly, the support shaft 314 also has a hollow structure so that the force transmission mechanism 304 can pass through. The diameter and material of the support shaft 314 may be the same as other parts of the instrument shaft 303, or may be different. One or more sensitive elements 315 are attached to the inner surface (preferably the inner circular surface) of the support shaft 314. The difference from the above embodiment is that the outer diameter of the terminal base 313 is smaller than the inner diameter of the support shaft 314, and slightly larger than the difference between the inner diameter of the support shaft 314 and the radial dimension of the sensitive element 315, that is, the terminal base 313 can be Inserted into the support shaft 314 in an interference fit manner. Similarly, the outer diameter of the terminal base 313 may be slightly smaller than the difference between the inner diameter of the support shaft 314 and the radial dimension of the sensitive element 315, that is, a clearance fit is obtained between the terminal base 313 and the support shaft 314, and a filler, such as an elastic rubber, silicone, or the like, are arranged in the clearance between the terminal base 313 and the support shafts 314. Preferably, the terminal base 313 is coaxially arranged and in a clearance fit manner with the support shaft 314.

In addition to the inner surface of the support shaft 314, the sensitive element 315 can also be attached to the outer surface (preferably the outer circular surface) of the terminal base 313. In this case, the outer diameter (i.e., the sum of the outer diameter of the terminal base 313 and the radial dimension of the sensitive element 315) of the terminal base 313 to which the sensitive element 315 has been attached is slightly larger than the inner diameter of the support shaft 314 to obtain an interference fit, or the outer diameter of the terminal base 313 and the radial dimension of the sensitive element 315 is slightly smaller than the inner diameter of the support shaft 314 to obtain a clearance fit, and a filler is used to fill the clearance between the two.

Either of the above terminal bases 310, 313 can be formed by the axial extension of the proximal end of the end effector 305.

In addition, the first connector and the second connector may be designed in one piece to simplify the structure of the surgical instrument and optimize the size of the surgical instrument. Similar to the above embodiment, in another preferred solution shown in Figs. 4a to 4c, the first connector is implemented as the outer layer structure 323 connected to the distal end of the body of the instrument shaft 303. The outer layer structure 323 has a hollow structure to facilitate the passage of the force transmission mechanism 304. The second connector is implemented as the inner layer structure 324 connected to the proximal end of the end effector 305, and the inner layer structure 324 is hollowed to facilitate the passage of the aforementioned force transmission mechanism 304. The connecting portion 320 is fixedly connected to the end effector 305, and preferably the inner layer structure 324 is fixedly connected to the end effector 305. In particular, the outer layer structure 323 is disposed at the periphery of the inner layer structure 324, and is radially connected to the inner layer structure 324, together forming a shaft arm of the connecting portion 320 of the instrument shaft. Thus, a groove 322 is formed between the inner layer structure 324 and the outer layer structure 323. Preferably, the groove 322 is U-shaped. Preferably, a reinforcing plate is provided in the groove 322 to enhance its structural strength. Furthermore, one or more sensitive elements 321 are disposed in the groove 322, for example, attached to the inner surface of the outer layer structure 323 of the connecting portion 320, or attached to the outer surface of the inner layer structure 324 of the connecting portion 320. Preferably, a filler is provided in the groove 322, such as elastic rubber, silicone, etc., so as to increase the elasticity of the connecting portion 320.

It should be understood that when the end effector 305 is subjected to the force exerted by the human tissue, the inner and outer layer structures forming the groove 322 may be deformed to some extent due to the hollow structure of the shaft arm of the connecting portion 320, thereby squeezing sensitive element 321, and accordingly the sensitive element 321 is deformed. According to the deformation, the pressure sensor unit can determine the force received by the sensitive element 321, and then can sense the force (including the magnitude and direction thereof) acting on the end effector 305, and finally determine the force acting on the terminal of the surgical instrument. Optionally, a plurality of the sensitive elements 321 are uniformly distributed circumferentially in the groove 322, and more preferably, the groove 322 is provided with a plurality of rows of sensitive elements 321 distributed circumferentially and uniformly along the axial direction, which improves the accuracy of detection.

While a detail description about the structures of the surgical instrument has been given above, it is a matter of course that the present application includes, but is not limited to, the above-described configurations, and any modification made thereto are intended to also fall within the scope of the application. Those skilled in the art can arrive at other embodiments in light of the teachings of the foregoing embodiments.

In addition, the present application also provides a surgical robot system. The surgical robot system includes a slave device. The slave device includes a robot arm and the surgical instrument mentioned above. The surgical instrument is detachably connected to the terminal of the robot arm, so that the surgical instrument can be driven to move around a remote center of motion. Furthermore, the surgical robot system further includes a master device and a control unit. The master device includes a force indicator. The control unit is communicatively connected with the master device and the slave device. The control unit is configured to determine the information of the Cartesian force received by the end effector from the sensitive element of the surgical instrument and transmit the information to the force indicator. Furthermore, the control unit 10 may employ an existing PLC controller, microcomputer, microprocessor or the like, and one skilled in the art will understand how to implement such a selection based on the disclosure herein in combination with the common general knowledge in the art.

The surgical instrument of the present application according to the present application has a first connector and a second connector that are radially distributed, and a sensitive element is disposed between the first connector and the second connector, and the sensitive element can sense the force acting on the terminal of the instrument shaft of the surgical instrument, and then the Cartesian force received by the end effector of the surgical instrument is determined. Furthermore, in some embodiments, the pressure sensor unit is a strain pressure sensor, a piezoresistive pressure sensor or a piezoelectric pressure sensor, and the sensitive element is arranged between the first connector and the second connector to sense the force acting on the connecting portion. The terminal of the surgical instrument is subjected to an external force, so that the first connector and the second connector are subjected to the force accordingly and transmit the force to the sensitive element, and thus the sensitive element is deformed. Such deformation is sensed by the sensitive element immediately, so that the force acting on the connecting portion can be determined. Therefore, the pressure between the first connector and the second connector is determined from the deformation information, and the Cartesian force acting on the terminal of the surgical instrument can be accurately and uniquely measured.

In particular, the distal end of the instrument shaft of the surgical instrument extends axially to form a double-layer hollow support shaft. Preferably, the support shaft has a groove with a U-shaped axial cross-section. Due to the U-shaped thin wall of the support shaft, the accuracy of determining the force acting on the terminal of the surgical instrument can be further improved.

Compared with the conventional solutions using a motor output to calculate the force acting on the terminal of the surgical instrument, the surgical instrument of the present application has advantages of both a simpler force transmission path and higher force measurement accuracy. Moreover, the force acting on the terminal of the surgical instrument can be determined in an easier manner independently of the constructions of the surgical instrument without requiring additional components, providing for lower structural complexity of the surgical instrument and facilitating its assembly. Further, since minor changes are required in the surgical instrument, various existing surgical instruments after being modified with minor changes can be suitably used in the surgical robot system proposed by the present application.

The above description only described the preferred embodiments of the present disclosure, and is not intended to limit the scope of the present disclosure. The scope of the invention is as defined by the appended claims.

## Claims

1. A surgical instrument (3), comprising a mechanical structural unit and a pressure sensor unit, wherein,
the mechanical structural unit comprises an instrument shaft (303) and an end effector (305); the instrument shaft (303) comprises a body and a connecting portion (320) extending from a distal end of the body; the connecting portion (320) comprises a first connector and a second connector that are coaxially disposed; the first connector is fixedly connected to the body of the instrument shaft (303), and the second connector is fixedly connected to the end effector (305);
the pressure sensor unit comprises a sensitive element (312, 315, 321) disposed between the first connector and the second connector;
**characterized in that**,
when the end effector (305) is subjected to Cartesian force, the second connector squeezes the sensitive element (312, 315, 321) so as to exert a force dependent on the Cartesian force onto the sensitive element (312, 315, 321), and the sensitive element (312, 315, 321) is configured to sense the force from the second connector thereby obtaining a force data so that the Cartesian force received by the end effector (305) is determined based on the force data.

2. The surgical instrument (3) of claim 1, wherein the first connector is a hollow support shaft (311), the second connector is a hollow base (310), the base (310) is formed from an axial extension of a proximal end of the end effector (305), and the base (310) is configured to surround the support shaft (311).

3. The surgical instrument (3) of claim 1, wherein the first connector is an outer layer structure (323), the second connector is an inner layer structure (324), the outer layer structure (323) is radially connected with the inner layer structure (324) to form a groove (322) in which the sensitive element (312, 315, 321) is arranged.

4. The surgical instrument (3) of claim 3, wherein the groove (322) has a U-shaped axial section.

5. The surgical instrument (3) of claim 1, wherein a an outer diameter of the first connector is smaller than an inner diameter of the second connector; and
the inner diameter of the second connector is slightly smaller than a sum of the outer diameter of the first connector and a radial dimension of the sensitive element (312, 315, 321) to obtain an interference fit.

6. The surgical instrument (3) of claim 1, wherein an inner diameter of the second connector is greater than a sum of an outer diameter of the first connector and a radial dimension of the sensitive element (312, 315, 321), and a filler is arranged between the first connector and the second connector to increase an elasticity between the first connector and the second connector.

7. The surgical instrument (3) of claim 1, wherein an inner diameter of the first connector is larger than an outer diameter of the second connector, and
the inner diameter of the first connector is slightly smaller than a sum of the outer diameter of the second connector and a radial dimension of the sensitive element (312, 315, 321) to obtain an interference fit.

8. The surgical instrument (3) of claim 1, wherein an inner diameter of the first connector is larger than a sum of an outer diameter of the second connector and a radial dimension of the sensitive element (312, 315, 321), and a filler is arranged between the first connector and the second connector to increase an elasticity between the first connector and the second connector.

9. The surgical instrument (3) of claim 1, wherein the pressure sensor unit comprises one sensitive element (312, 315, 321), or comprises a plurality of sensitive elements (312, 315, 321), and the plurality of sensitive elements (312, 315, 321) are distributed along a circumferential direction and/or an axial direction of the surgical instrument (3).

10. The surgical instrument (3) of claim 9, wherein the plurality of sensitive elements (312, 315, 321) are distributed in a plurality of rows in the axial direction of the surgical instrument (3), and the sensitive elements (312, 315, 321) in each of the rows are evenly distributed in the circumferential direction of the surgical instrument (3).

11. The surgical instrument (3) of claim 10, wherein each of the rows of sensitive elements (312, 315, 321) is arranged staggeredly with an adjacent one of the rows of sensitive elements (312, 315, 321).

12. A surgical robot system, comprising a slave device comprising:
a robotic arm (2); and the surgical instrument (3) of any one of claims 1 to 11;
wherein the robotic arm (2) has a terminal detachably connected to the surgical instrument (3), and is configured to drive the surgical instrument (3) to pivot about a remote center of motion.

13. The surgical robot system of claim 12, further comprising a master device and a control unit (10), the master device comprising a force indicator;
wherein the control unit (10) is communicatively connected to the master device and to the slave device, and the control unit (10) is configured to obtain information about a Cartesian force received by the end effector (305) from the sensitive element (312, 315, 321) of the surgical instrument (3) and transmits the information to the force indicator.

14. The surgical robot system of claim 13, wherein the force indicator is a master manipulator (6) provided with a motor, and the control unit (10) is configured to issue a torque command to the motor of the master manipulator (6) to enable an operator to feel a force acting on the terminal end of the surgical instrument (3).

15. The surgical robot system of claim 14, wherein the master manipulator (6) further comprises a vibrating motor; when the force acting on the terminal end of the surgical instrument (3) exceeds a preset threshold, the control unit (10) issues a vibration command to the vibrating motor of the master manipulator (6), notifying the operator about the excessive force acting on the terminal end of the surgical instrument (3).

## Patentansprüche

1. Chirurgisches Instrument (3), umfassend eine mechanische Struktureinheit und eine Drucksensoreinheit, wobei
die mechanische Struktureinheit einen Instrumentenschaft (303) und einen Endeffektor (305) umfasst; der Instrumentenschaft (303) einen Körper und einen Verbindungsabschnitt (320) umfasst, der sich von einem distalen Ende des Körpers erstreckt; der Verbindungsabschnitt (320) einen ersten Verbinder und einen zweiten Verbinder umfasst, die koaxial angeordnet sind; der erste Verbinder fest mit dem Körper des Instrumentenschafts (303) verbunden ist und der zweite Verbinder fest mit dem Endeffektor (305) verbunden ist;
die Drucksensoreinheit ein empfindliches Element (312, 315, 321) umfasst, das zwischen dem ersten Verbinder und dem zweiten Verbinder angeordnet ist;
**dadurch gekennzeichnet, dass**
wenn der Endeffektor (305) einer kartesischen Kraft ausgesetzt ist, der zweite Verbinder das empfindliche Element (312, 315, 321) zusammendrückt, um auf das empfindliche Element (312, 315, 321) eine von der kartesischen Kraft abhängige Kraft auszuüben, und das empfindliche Element (312, 315, 321) ausgebildet ist, die Kraft von dem zweiten Verbinder zu erfassen und dadurch Kraftdaten zu erhalten, so dass die von dem Endeffektor (305) empfangene kartesische Kraft basierend auf den Kraftdaten bestimmt wird.

2. Chirurgisches Instrument (3) nach Anspruch 1, wobei der erste Verbinder ein hohler Stützschaft (311) ist, der zweite Verbinder eine hohle Basis (310) ist, die Basis (310) aus einer axialen Verlängerung eines proximalen Endes des Endeffektors (305) gebildet ist und die Basis (310) ausgebildet ist, den Stützschaft (311) zu umgeben.

3. Chirurgisches Instrument (3) nach Anspruch 1, wobei der erste Verbinder eine äußere Schichtstruktur (323) ist, der zweite Verbinder eine innere Schichtstruktur (324) ist, die äußere Schichtstruktur (323) radial mit der inneren Schichtstruktur (324) verbunden ist, um eine Nut (322) zu bilden, in der das empfindliche Element (312, 315, 321) angeordnet ist.

4. Chirurgisches Instrument (3) nach Anspruch 3, wobei die Nut (322) einen U-förmigen axialen Abschnitt aufweist.

5. Chirurgisches Instrument (3) nach Anspruch 1, wobei ein Außendurchmesser des ersten Verbinders kleiner als ein Innendurchmesser des zweiten Verbinders ist; und
der Innendurchmesser des zweiten Verbinders etwas kleiner als die Summe des Außendurchmessers des ersten Verbinders und einer radialen Abmessung des empfindlichen Elements (312, 315, 321) ist, um eine Presspassung zu erhalten.

6. Chirurgisches Instrument (3) nach Anspruch 1, wobei ein Innendurchmesser des zweiten Verbinders größer als die Summe aus einem Außendurchmesser des ersten Verbinders und einer radialen Abmessung des empfindlichen Elements (312, 315, 321) ist, und wobei ein Füllstoff zwischen dem ersten Verbinder und dem zweiten Verbinder angeordnet ist, um eine Elastizität zwischen dem ersten Verbinder und dem zweiten Verbinder zu erhöhen.

7. Chirurgisches Instrument (3) nach Anspruch 1, wobei ein Innendurchmesser des ersten Verbinders größer als ein Außendurchmesser des zweiten Verbinders ist, und
der Innendurchmesser des ersten Verbinders etwas kleiner als die Summe aus dem Außendurchmesser des zweiten Verbinders und einer radialen Abmessung des empfindlichen Elements (312, 315, 321) ist, um eine Presspassung zu erhalten.

8. Chirurgisches Instrument (3) nach Anspruch 1, wobei ein Innendurchmesser des ersten Verbinders größer als die Summe aus einem Außendurchmesser des zweiten Verbinders und einer radialen Abmessung des empfindlichen Elements (312, 315, 321) ist, und ein Füllstoff zwischen dem ersten Verbinder und dem zweiten Verbinder angeordnet ist, um die Elastizität zwischen dem ersten Verbinder und dem zweiten Verbinder zu erhöhen.

9. Chirurgisches Instrument (3) nach Anspruch 1, wobei die Drucksensoreinheit ein empfindliches Element (312, 315, 321) umfasst oder eine Vielzahl von empfindlichen Elementen (312, 315, 321) umfasst und die Vielzahl von empfindlichen Elementen (312, 315, 321) entlang einer Umfangsrichtung und/oder einer Axialrichtung des chirurgischen Instruments (3) verteilt sind.

10. Chirurgisches Instrument (3) nach Anspruch 9, wobei die Vielzahl von empfindlichen Elementen (312, 315, 321) in einer Vielzahl von Reihen in der axialen Richtung des chirurgischen Instruments (3) verteilt sind und die empfindlichen Elemente (312, 315, 321) in jeder der Reihen gleichmäßig in der Umfangsrichtung des chirurgischen Instruments (3) verteilt sind.

11. Chirurgisches Instrument (3) nach Anspruch 10, wobei jede der Reihen von empfindlichen Elementen (312, 315, 321) versetzt zu einer benachbarten Reihe von empfindlichen Elementen (312, 315, 321 ) angeordnet ist.

12. Chirurgisches Robotersystem, das ein Slave-Gerät umfasst, welches umfasst:
einen Roboterarm (2); und das chirurgische Instrument (3) nach einem der Ansprüche 1 bis 11;
wobei der Roboterarm (2) einen Anschluss aufweist, der abnehmbar mit dem chirurgischen Instrument (3) verbunden ist, und ausgebildet ist, das chirurgische Instrument (3) anzutreiben, um einen entfernten Bewegungsmittelpunkt zu schwenken.

13. Chirurgisches Robotersystem nach Anspruch 12, ferner umfassend ein Master-Gerät und eine Steuereinheit (10), wobei das Master-Gerät einen Kraftindikator umfasst;
wobei die Steuereinheit (10) kommunikativ mit dem Master-Gerät und dem Slave-Gerät verbunden ist, und die Steuereinheit (10) ausgebildet ist, Informationen über eine vom empfindlichen Element (312, 315, 321) des chirurgischen Instruments (3) durch den Endeffektor (305) empfangene kartesische Kraft zu erhalten und die Informationen an den Kraftindikator zu übertragen.

14. Chirurgisches Robotersystem nach Anspruch 13, wobei der Kraftindikator ein mit einem Motor ausgestatteter Hauptmanipulator (6) ist und die Steuereinheit (10) ausgebildet ist, einen Drehmomentbefehl an den Motor des Hauptmanipulators (6) auszugeben, um es einem Bediener zu ermöglichen, eine auf das Abschlussende des chirurgischen Instruments (3) wirkende Kraft zu spüren.

15. Chirurgisches Robotersystem nach Anspruch 14, wobei der Hauptmanipulator (6) einen Vibrationsmotor ferner umfasst; wenn die auf das Abschlussende des chirurgischen Instruments (3) wirkende Kraft einen voreingestellten Schwellenwert überschreitet, gibt die Steuereinheit (10) einen Vibrationsbefehl an den Vibrationsmotor des Hauptmanipulators (6) aus, um den Bediener über die übermäßige Kraft zu informieren, die auf das Abschlussende des chirurgischen Instruments (3) wirkt.

## Revendications

1. Instrument chirurgical (3), comprenant une unité structurelle mécanique et une unité de capteur de pression, dans lequel,
L'unité structurelle mécanique comprend une tige d'instrument (303) et un effecteur d'extrémité (305) ; la tige d'instrument (303) comprend un corps et une partie de connexion (320) s'étendant à partir d'une extrémité distale du corps ; la partie de connexion (320) comprend un premier connecteur et un second connecteur qui sont disposés de manière coaxiale ; le premier connecteur est connecté de manière fixe au corps de la tige d'instrument (303) et le second connecteur est connecté de manière fixe à l'effecteur d'extrémité (305) ;
l'unité de capteur de pression comprend un élément sensible (312, 315, 321) disposé entre le premier connecteur et le deuxième connecteur ;
**caractérisé en ce que**,
lorsque l' effecteur terminal (305) est soumis à une force cartésienne , le deuxième connecteur comprime l' élément sensible (312, 315, 321) de manière à exercer une force dépendant du plan cartésien force sur l'élément sensible (312, 315, 321), et l'élément sensible (312, 315, 321) est configuré pour détecter la force provenant du second connecteur , obtenant ainsi des données de force de sorte que la force cartésienne reçue par l'effecteur d'extrémité (305) soit déterminée sur la base des données de force.

2. Instrument chirurgical (3) selon la revendication 1, dans lequel le premier connecteur est une tige de support creuse (311), le second connecteur est une base creuse (310), la base (310) est formée à partir d'une extension axiale d'une extrémité proximale de l'effecteur terminal (305), et la base (310) est configurée pour entourer la tige de support (311).

3. Instrument chirurgical (3) selon la revendication 1, dans lequel le premier connecteur est une structure de couche externe (323), le second connecteur est une structure de couche interne (324), la structure de couche externe (323) est reliée radialement à la structure de couche interne (324) pour former une rainure (322) dans laquelle l'élément sensible (312, 315, 321) est disposé.

4. Instrument chirurgical (3) selon la revendication 3, dans lequel la rainure (322) a une section axiale en forme de U.

5. Instrument chirurgical (3) selon la revendication 1, dans lequel un diamètre extérieur du premier connecteur est inférieur à un diamètre intérieur du second connecteur ; et
le diamètre intérieur du deuxième connecteur est légèrement inférieur à la somme du diamètre extérieur du premier connecteur et d'une dimension radiale de l'élément sensible (312, 315, 321) pour obtenir un ajustement serré.

6. Instrument chirurgical (3) selon la revendication 1, dans lequel un diamètre intérieur du second connecteur est supérieur à une somme d'un diamètre extérieur du premier connecteur et d'une dimension radiale de l'élément sensible (312, 315, 321), et une charge est disposée entre le premier connecteur et le second connecteur pour augmenter une élasticité entre le premier connecteur et le second connecteur.

7. Instrument chirurgical (3) selon la revendication 1, dans lequel un diamètre intérieur du premier connecteur est plus grand qu'un diamètre extérieur du second connecteur, et
le diamètre intérieur du premier connecteur est légèrement inférieur à la somme du diamètre extérieur du deuxième connecteur et d'une dimension radiale de l'élément sensible (312, 315, 321) pour obtenir un ajustement serré.

8. Instrument chirurgical (3) selon la revendication 1, dans lequel un diamètre intérieur du premier connecteur est supérieur à une somme d'un diamètre extérieur du second connecteur et d'une dimension radiale de l'élément sensible (312, 315, 321), et une charge est disposée entre le premier connecteur et le second connecteur pour augmenter une élasticité entre le premier connecteur et le second connecteur.

9. Instrument chirurgical (3) selon la revendication 1, dans lequel l'unité de capteur de pression comprend un élément sensible (312, 315, 321), ou comprend une pluralité d'éléments sensibles (312, 315, 321), et la pluralité d'éléments sensibles (312, 315, 321) sont répartis le long d'une direction circonférentielle et/ou d'une direction axiale de l'instrument chirurgical (3).

10. Instrument chirurgical (3) selon la revendication 9, dans lequel la pluralité d'éléments sensibles (312, 315 , 321) sont répartis dans une pluralité de rangées dans la direction axiale de l' instrument chirurgical (3), et les éléments sensibles (312, 315, 321) dans chacune des rangées sont répartis uniformément dans la direction circonférentielle de l'instrument chirurgical (3).

11. Instrument chirurgical (3) selon la revendication 10, dans lequel chacune des rangées d'éléments sensibles (312, 315, 321) est disposée en quinconce avec une rangée adjacente des éléments sensibles (312, 315, 321).

12. Système de robot chirurgical, comprenant un dispositif esclave comprenant :
un bras robotisé (2) ; et l'instrument chirurgical (3) selon l'une quelconque des revendications 1 à 11 ;
dans lequel le bras robotique (2) comporte un terminal relié de manière détachable à l'instrument chirurgical (3), et est configuré pour entraîner l'instrument chirurgical (3) à pivoter autour d'un centre de mouvement distant.

13. Système de robot chirurgical selon la revendication 12, comprenant en outre un dispositif maître et une unité de commande (10), le dispositif maître comprenant un indicateur de force ;
dans lequel l'unité de commande (10) est connectée de manière communicative au dispositif maître et au dispositif esclave, et l'unité de commande (10) est configurée pour obtenir des informations sur une force cartésienne reçue par l'effecteur terminal (305) à partir de l'élément sensible (312, 315, 321) de l'instrument chirurgical (3) et transmet les informations à l'indicateur de force.

14. Système de robot chirurgical selon la revendication 13, dans lequel l'indicateur de force est un manipulateur maître (6) pourvu d'un moteur, et l'unité de commande (10) est configurée pour émettre une commande de couple au moteur du manipulateur maître (6) pour permettre à un opérateur de ressentir une force agissant sur l' extrémité terminale de l'instrument chirurgical (3).

15. Système de robot chirurgical selon la revendication 14, dans lequel le manipulateur maître ( 6) comprend en outre un moteur vibrant ; lorsque la force agissant sur l' extrémité terminale de l'instrument chirurgical (3) dépasse un seuil prédéfini, l'unité de commande (10) émet une commande de vibration au moteur vibrant du manipulateur maître (6), notifiant à l'opérateur la force excessive agissant sur l' extrémité terminale de l'instrument chirurgical (3).
